# EUROPEAN PATENT SPECIFICATION

(11) **EP 2 231 174 B1**
(45) Date of publication and mention of the grant of the patent: **13.02.2013**
(21) Application number: 08865377.9
(22) Date of filing: 22.12.2008
(51) Int. Cl.: A61K 36/15, A23L 1/30, A61P 1/12, A61P 15/02, A61P 17/00

(54) **USE LARCH WOOD MATERIAL FOR TREATING INFLAMMATION**
VERWENDUNG VON LÄRCHENHOLZMATERIAL ZUR BEHANDLUNG VON ENTZÜNDUNGEN
APPLICATION D'UNE MATIÈRE À BASE DE BOIS DE MÉLÈZE POUR DES APPLICATIONS MÉDICALES

(30) Priority: 21.12.2007 EP 07450242
(43) Date of publication of application: 29.09.2010
(73) Proprietor: University of Graz, 8010 Graz (AT); University of Udine, 33100 Udine (IT); Aristotle University Of Thessaloniki, 54124 Thessaloniki (GR); University of Veterinary Medicine Vienna, 1210 Vienna (AT)
(72) Inventor: BAUER, Rudolf, A-8042 Graz (AT); WENZIG, Eva-Maria, A-8010 Graz (AT); FRANZ, Chlodwig, A-1190 Vienna (AT); STEFANON, Bruno, I-33100 Udine (IT); TZIKA, Eleni, GR-54454 Thessaloniki (GR); KYRIAKIS, Spiros, GR-19009 Rafina (GR); FARINACCI, Maura, I-33100 Udine (IT); SGORLON, Sandy, I-33050 Mortegliano (IT)
(74) Representative: Patentanwaltskanzlei Matschnig & Forsthuber OG
(86) International application number: PCT/AT2008/000473
(87) International publication number: WO 2009/079680

(56) References cited:
- EP-A- 0 939 771
- WO-A-96/03150
- WO-A-98/27829
- WO-A-2005/047423
- DATABASE WPI Week 200043 Thomson Scientific, London, GB; AN 2000-489765 XP002528972 & RU 2 141 317 C1 (LOPAREV P I) 20 November 1999 (1999-11-20)
- DATABASE WPI Week 200540 Thomson Scientific, London, GB; AN 2005-393454 XP002528973 & RU 2 252 028 C2 (A MED E SIBE SURGERY RES CENTRE) 20 May 2005 (2005-05-20)

## Description

The invention relates to the use of raw larch wood material, primarily larch sawdust in the manufacture of a medicament for treating/preventing inflammation.

Larches are conifers in the genus *Larix* (family Pinaceae). The genus comprises about 10 commonly recognized species and a range of cultivars and varieties. Members of the genus Larix are widely distributed across Europe, North America and Asia. Larch resources play an economically and ecologically important role in the Northern Hemisphere. The European Larch, *Larix decidua* MILL. (Pinaceae), which the present invention is primarily based on, is an up to 35 m high, deciduous tree, which is mainly native to the higher regions of the Alps, Sudetes and Carpathian mountains.

Larch wood is tough, waterproof and durable, but also flexible in thin strips. The heartwood is particularly weather-proof and is, therefore, mainly used as construction timber. Due to its high content of resin and difficult machinability it is hardly used for fabricating furniture.

The present invention is primarily focused on the exploitation of larch sawdust. Larch sawdust is a waste product in wood industry. At present it is mainly used as a fuel material. The material is cheap and easily available in high amounts. As it is a waste product, the exploitation of this material for medical purposes would significantly enhance its value.

Larch wood is known to contain lignans, flavonols, polysaccharides and oleoresin. Larch resin (Terebinthina laricina) consists of about 15% essential oils , 50 to 65% resin acids and about 15% non-saponifiable resin. Main constituent of the neutral fraction is larixyl acetate, apart from larixol and 13-epimanool. In the neutral fraction also diterpene acids like dehydroabietic acid, isopimaric acid, palustric acid, abietic acid and neoabietic acid are found. It has been found that the oleoresin of *Larix decidua* MILL. contains remarkably high amounts of larixyl acetate. Applications for larch wood phytochemicals, in particular the polysaccharide arabinogalactan, are found in the food, pharmaceutical and cosmetic industries. Larch arabinogalactan, a water-soluble polysaccharide deriving mainly from *Larix occidentalis,* is approved by the FDA as a source of dietary fiber, but has also potential therapeutic effects as an immunostimulant and cancer protocol adjunct. The phenolic constituents of larch wood are known to possess good antioxidant activity.

EP 0 939 771 B1 discloses lipidated arabinogalactan compositions for a wide variety of different biomedical applications including the inhibition of infection or inflammation. The compositions are preferentially administered parenterally. Due to arabinogalactan's high molecular weights - major fractions in larch are 16 000 to 100 000 Da - and its polar character, resorption by the body after oral administration is very unlikely. Consequently, when administered orally, e.g. as a feed additive, a sufficient overall anti-inflammatory effect cannot be expected.

The flavonoids dihydroquercetin and dihydrokaemferol are known to inhibit protein kinase C and the expression of several pro-inflammatory genes. However, almost the same as for arabinogalactan applies for these larch phytochemicals. Sufficient resorption by the body and a resultant overall anti-inflammatory effect after oral administration is very unlikely due to their polar character.

The WO 98/27829 A1 discloses a food additive and a preparation method thereof for the prevention of gastrointestinal disorders and promotion of growth, wherein hydrolytically treated raw material, including larch among others, is used. The EP 0 797 451 B1 discloses compositions containing hemicelluloses in combination with polyphenols from Larix for use as a feed additive for treatment of gastrointestinal disorders.

Diterpenes are constituents of natural resins, such as colophony resin, which is gained from conifer trees like spruce, firs and pines. Larches belong to the family *Pinaceae,* as already mentioned.

The document DE 31 13 460 A1 discloses a pharmaceutical composition for treatment of chronic disorders such as arthritis and rheumatism, wherein the preparation contains a mixture of resin acids, including the diterpenes laevopimaric acid, neoabietic acid, palustric acid, abietic acid, pimaric acid and isopimaric acid. The composition is most effective when applied topically, but might also be applied orally. Nevertheless, this composition is not useful as a feed supplement (feed additive) in animal husbandry, since a large quantity is required and production thereof would be far too expensive. A topical application providing the desired effect would be very time-consuming and awkward (e.g. treatment of mastitis in cows). In addition, an extraction of larch wood material would lead to large amounts of solvent-contaminated material which has to be disposed professionally.

Another constituent of larch wood, abietic acid, is known to show a medium-pronounced 5-lipoxygenase (5-LOX) inhibitory activity with an IC₅₀ of ~ 30 µM. 5-LOX is crucial in the formation of a group of biologically active lipids referred to as leukotrienes, which play important roles in the pathophysiology of a variety of inflammatory and allergic responses. A more pronounced 5-LOX inhibitory activity, however, would be beneficial. In *Larix decidua* MILL. abietic acid is contained only to a minor degree. The inventors determined a content of 0.001-0.005 %in larch sawdust. Moreover, the production of a feed supplement (feed additive) based on an abietic acid-extract of larch sawdust would be too expensive. As for the composition in DE 31 13 460 A1 an extraction would lead to large amounts of solvent-contaminated material, which has to be professionally disposed.

Beside lipoxygenases, cyclooxygenases (COX) are key enzymes in the metabolism of arachidonic acid to oxygenated arachidonic acid metabolites, the so-called prostanoids, some of which play an important role in inflammatory processes, fever, thromboses, allergic and immune reactions as well as cancer. The cyclooxygenase pathway leads to the production of thromboxane A2, prostacyclin and prostaglandins. In the lipoxygenase pathway, arachidonic acid is converted to leukotrienes and lipoxins. 5-LOX may be the biologically most important lipoxygenase, as it leads to the synthesis of leukotrienes. Leukotrienes (LTs) have been shown to play an important role in the pathogenesis of inflammatory diseases such as arthritis, psoriasis, inflammatory bowel disease, and asthma. This finding led to the development of leukotriene inhibitors for the treatment of allergic and inflammatory diseases. These drugs either act as LT receptor antagonists or as inhibitors of LT biosynthesis. Concerning the second mechanism of action, only one drug has reached the market up to now, namely Zileuton which is used for treatment of asthma in the USA. COX exist in at least two different isoforms, one of which (COX-1) has been regarded as the constitutive enzyme possessing housekeeping and cytoprotective functions. COX-2 is highly expressed in inflammation-related cell types such as macrophages and mast cells after stimulation with proinflammatory agents. Therefore, this isoform was associated with inflammatory diseases. The conclusion was drawn that many side effects observed in nonsteroidal anti-inflammatory drugs (NSAIDs) such as gastrointestinal damage and platelet dysfunction are due to the inhibition of COX-1 derived prostanoids whereas the inhibition of COX-2 derived prostaglandins leads to anti-inflammatory, antipyretic and analgesic effects. This led to the development of a series of highly selective COX-2 inhibitors. However, recently it turned out that certain highly selective COX-2 inhibitors may increase the risk of side effects such as cardiovascular diseases, a phenomenon which is still intensively investigated; furthermore, it was found that also COX-2 is expressed constitutively in certain tissues and not only involved in inflammatory processes.

According to the current opinion [see Editorial/Joint Bone Spine 72 (2005): 199-201] in inflammation treatment, the dual inhibition of both cyclooxygenases, COX-1 and COX-2, and 5-LOX is a novel therapeutic approach to inflammation, suggesting drugs with no prothrombotic effect and a good gastrointestinal safety profile.

WO 96/03150 A discloses compositions comprising dietary fibres and polyphenols isolated from Larix trees for treating inflammation in humans and animals. RU 2141 317 C1 discloses the use of aqueous extracts of larch wood bark for treating gastrointestinal disorders in animals.

It is therefore an object of the present invention to exploit larch wood material, preferably the waste-product larch sawdust, for medical purposes.

It is a further object of the present invention to provide an inexpensive anti-inflammatory agent, particularly in form of a feed additive, in animal husbandry.

The present invention refers to the use of raw wood material from a tree of the genus *Larix* in the manufacture of a medicament for treatment or prophylaxis of inflammation in humans or animals.

According to the present invention "larch wood material" refers to all kind of raw wood material obtained from a tree of the genus Larix. Preferably the larch wood material used within the scope of this invention derives from *Larix decidua* MILL. The larch wood material can, however, derive from other members of the genus *Larix* as well. Preferably the larch wood material is larch sawdust, which is a waste product in wood industry. It is inexpensive and easily available in high amounts. The term "larch sawdust" also refers to larch wood shavings. Other kinds of waste wood from larch (e.g. defective or wrongly tailored wooden planks, wastes accruing in woodcutting, scrap wood) can also be used within the frame of the present invention.

In order to detect potential anti-inflammatory properties, extracts from larch sawdust of *Larix decidua* MILL. were investigated in several *in vitro* experimental models (see also Example 1). Larch sawdust in form of the raw material was investigated in feeding trials (see also Examples 2 and 3). The inventors could demonstrate that the raw material and extracts thereof possess a strong anti-imflammatory effect. It turned out that the heptane extract from sawdust of *Larix decidua* Mill. possesses a strong *in vitro* inhibitory activity against cyclooxygenase-1 (COX-1), cyclooxygenase-2 (COX-2), and 5-lipoxygenase (5-LOX) mediated leukotriene (LT) biosynthesis (see Example 1 stated below in the description). Diterpenes isolated from the heptane extract have been shown to possess LT biosynthesis inhibitory activity to different extents and moderate COX-2 inhibitory activity (for detailed results see also Example 1). The degree of inhibitory activity of larch extracts is comparable to the activity of compounds (see Example 1, Table 6: Indomethacin, NS-398, Zileuton), which are already established as therapeutics against inflammatory disorders and asthma. This is especially remarkable in view of the fact that the heptane extract is a crude extract. The heptane extract contains lipophilic compounds comprising neutral labdane diterpenoids as well as abietane diterpenoids. Larixyl acetate is contained to a considerable extent in both raw material and extract (typically from 0.04 to 0-07% in the raw material and from 5 to 12% in the dry extract). Larixyl acetate showed an unexpected strong LT biosynthesis inhibitory activity comparable to the established 5-LOX inhibitor Zileuton and weak COX-2 activity. A pronounced LT biosynthesis inhibitory activity was also observed for dehydroabietinol and isopimaric acid, however, both compounds are contained only to a very low extent in larch wood. Dehydroabietic acid (included typically from 0.01 to 0.025% in the raw material and 2.5 to 4.5% in the dry extract), having a structure similar to abietic acid, showed only weak LT biosynthesis inhibitory activity. According to these findings, a strong LT biosynthesis inhibitory activity contributing to an unexpected strong anti-inflammatory effect may be attributed to larixyl acetate. In conclusion, these unexpected findings open new ways in exploiting raw larch wood material, for treating and preventing inflammation.

In contrast to the state of the art mentioned above (see EP 0 939 771 B1 and the flavonoids dihydroquercetin and dihydrokaemferol), all diterpene compounds are easily resorbed by the body after oral application due to their lipophilic character. According to the invention, the diterpene compounds are applied in form of raw larch wood material. The diterpene compounds in the raw larch wood material are well and sufficiently resorbed by the body resulting in an overall anti-inflammatory activity, which is not merely confined to the gastrointestinal tract. As already mentioned above, the inhibition of all three enzymes COX-1, COX-2 and 5-LOX is considered as a great advantage in treating inflammation not only efficiently but also in a safe way. In view of these unexpected findings, it is therefore advantageous to use larch wood material, preferably inexpensive larch sawdust, for treating or preventing inflammation.

According to the invention, the larch wood material is preferably administered orally, since oral administration is easy to perform and little time-consuming. The use of larch wood material is particularly useful in animal husbandry, as a large number of individuals can be addressed in this way in a time-saving manner. In one specific aspect it may be used as an anti-inflammatory ingredient in a preparation of a nutritional composition (functional food) for humans and animals.

As stated above, according to the invention the larch wood material is used in form of raw material, since the production of therapeutic agents based on larch wood extracts, especially in view of animal husbandry, can be very time-consuming and very expensive. Apart from the above-mentioned reasons, the use of raw material rather than extracts thereof is also favourable with regard to environmental/ecological consciousness and awareness, since an extraction from larch wood would lead to large amounts of solvent-contaminated wood material, which will require professional disposal. *In vitro* results with larch sawdust extracts (see Example 1 below) and preliminary results obtained from feeding trials (see Examples 2 and 3 below) with raw material of larch sawdust showed very good anti-inflammatory efficacy.

The water content of the larch wood material does not influence its anti-inflammatory efficacy. Drying the wood material during manufacture of an anti-inflammatory medicament based on larch wood material, however, may be necessary in order to obtain reproducible and standardized products.

The present invention is particularly useful in animal husbandry and provides an anti-inflammatory medicament for farm animals, which include, but are not limited to ruminants (sheep and cattle), swine and poultry. Among farm animals, larch wood material is preferably used for ruminant species, considering that the adverse effect of fibre and lignin for the digestibility of the feedstuffs is minimized. The use of larch can be foreseen also for other herbivores, such as rodents and horses; also for these species the fibrous nature of the larch does not lead to adverse effects on ration digestibility. Furthermore, the invention is useful for treating or preventing inflammation in companion animals, which include, but are not limited to dogs and cats. The use of low doses of raw waste does not have any particular side effects for digestion in nonherbivore monogastric animals.

The term "inflammation" in this disclosure refers to non-infectious inflammatory conditions, but can also relate to all the infectious diseases and conditions known to those skilled in the art, where an increase of inflammation is expected. Since the active compounds are well resorbed by the human or animal body after oral application, the use of the invention is not limited to inflammation occurring in the gastrointestinal tract. The invention is particularly useful in treating inflammatory conditions, wherein the inflammation is measurable in blood. Inflammation markers measurable in blood are e.g. proinflammatory cytokines including Tumor Necrosis Factor-α (TNF-α), interleukin-1, interleukin-6, interleukin-2 and interferon-γ (type II interferons).

Mastitis, vaginitis, arthritis and osteoarthritis, cervicitis, metritis, endomeiritis, retained placenta, wound infection, uretritis, dermatitis, eczema, gastroenteritis, colitis, and diarrhea are examples, not exclusive, of the inflammatory diseases commonly encountered in the veterinary practice. Inflammatory conditions not associated with infection are very common in dairy cows around parturition, in the period referred as "transition cow" that comprises the interval from 30 days before to 30 days after calving. Other non-infectious inflammatory conditions in domestic animals are those related to environmental stress, where the secretion of cortisol leads to an increase of its blood concentration higher than tenfold the basal value. Similar situations can occur after farm animal transportation, modification of herd hierarchy, isolation of animals or during weaning, for mammals. This latter condition is well known for piglets, which underwent an early-weaning programme. Osteoarthrosis is a very common disease for sports and companion animals such as riding horses and dogs. Often, these types of disease are inherited, but are exacerbated by intensive training or physical exercise, unbalanced diet and related obesity.

As a result of the good anti-inflammatory efficacy of larch wood material, a beneficial effect with regard to growth promotion in animal husbandry may be expected.

Due to the observed anti-inflammatory effects, larch wood material may also be used as an anti-inflammatory agent, e.g. as an ingredient in a nutritional composition (functional food) to prevent or treat a variety of inflammatory diseases in humans like rheumatic diseases, asthma, colitis and psoriasis.

In order to obtain good anti-inflammatory efficacy, preferably wood material reduced to small pieces is used, wherein at least 95% by weight has a size less than or equal to 3 mm. The term "reduced to small pieces" refers to larch wood material chopped or pulverized; most preferably, the larch wood material, as already mentioned above, is larch sawdust. The term "larch sawdust" may also refer to larch wood shavings. Waste wood from larch (e.g. defective or wrongly tailored wooden planks, wastes accruing in woodcutting, scrap wood) can also be used as starting material within the frame of the present invention. The smaller the particle size, the better the anti-inflammatory effect. Also for ruminants, particle sizes may be less than or equal to 3 mm, to minimize the degradability in the rumen. The rumen outflow for small particles is faster, allowing the larch to express its action in the lower digestive tract. Preferably, when adding larch material to loose feed, the particle size should not be smaller than 0,5 mm in order to obtain a good homogenous distribution of the larch material in the feed.

More preferably, at least 95% by weight of the wood material has a size between 0.5 and 1.5 mm, inclusive. In this way, a good anti-inflammatory effect and an optimal homogeneous distribution is obtained. This variant is particularly useful for both ruminant and monogastric farm animals, such as swine and poultry (see Examples 2 and 3 as mentioned below).

According to another development of the invention, the wood material is preferably pulverized, wherein at least 95% by weight has a size between 0.05 and 0.5 mm, inclusive. This development is particularly useful as an ingredient in complete food, wherein the food is provided in the form of pressed food or flakes (e.g. food for domestic animals like cats or dogs). Moreover, pulverized sawdust can be used as an anti-inflammatory ingredient in a preparation of a nutritional composition to be given to humans, for example as an ingredient of a cereal bar, cereal flakes or bread.

The use of the wood material can be in a food supplement (food additive), wherein the term "food" particularly refers to animal feed, but also includes food for human individuals. The food supplement is preferably provided in form of dosage units and most preferably as a package with a predefined filling quantity. The wood material may represent up to 100% by weight of the food supplement. Preferably the amount of wood material may represent 70 - 99 % by weight in said food supplement, wherein the residual portion represents carriers and/or excipients. Typically, carriers are edible, e.g. dry or liquid food, or other compounds or compositions intended for nutritional purposes. Excipients are typically pharmaceutically acceptable for oral application, e.g. incorporation of larch wood material with excipients such as in form of tablets, pills or capsules, wherein excipients can be binding agents, siccatives or coatings.

The administration of the wood material can be specifically adapted to the respective individual, to which the wood material is dispensed, according to the present invention, animals and humans.

In one further aspect, the wood material or the food supplement is administered to ruminants, and preferably is in the form of an ingredient designed to be added to a food ration or a daily food intake given to ruminants or to be embedded as an ingredient during the manufacture of a complete food at a rate of from 0.5 to 10% by weight relative to the total weight of the dry matter food intake or of the complete food. According to the inventors' findings, larch sawdust can be included to an amount of 10% dry matter of daily ration without affecting intake and rumen activity. Most preferably larch sawdust is included at a rate of from 2 to 5% by weight relative to the total weight of the dry matter food intake. In case the food supplement also contains carriers and/or excipients, the food supplement can be added up to 10% by weight relative to the total weight of the dry matter food intake or of the complete food, wherein the amount of raw larch material should not exceed 7% by weight of the dry matter food intake or of the complete food. As already disclosed above, the particle size of larch wood material added to loose food for ruminants may be in the range from 0.5 up to 3 mm in order to ensure good anti-inflammatory efficacy as well as good homogeneous distribution of the larch wood material in the diet.

In another aspect, the wood material or the food supplement is administered to non-ruminant, herbivore farm animals, particularly rabbits and horses. The wood material or the food supplement, respectively, is preferably in the form of an ingredient designed to be added to a food ration or a daily food intake given to non-ruminant, herbivore farm animals or to be embedded as an ingredient during the manufacture of a complete food at a rate of from 0.1 to 2% by weight relative to the total weight of the food intake or of the complete food. The raw material, having preferably a particle size distribution between 0.5 and 1.5 mm, is well accepted at an inclusion level of up to 1.5 % by weight, most preferably at 1% by weight, in the diet. In case the food supplement also contains carriers and/or excipients, the food supplement can be added up to 2% by weight relative to the total weight of the food intake or of the complete food, wherein the amount of raw larch material should not exceed 1.5% by weight of the food intake or of the complete food.

In yet another aspect, the wood material or the food supplement is administered to non-herbivore farm animals and domestic animals, particularly swine, poultry, cats, dogs and rodents; preferably it is in the form of an ingredient designed to be added to a food ration or a daily food intake given to the animals or to be embedded as an ingredient during the manufacture of a complete food at a rate of from 0.1 to 1.5% by weight relative to the total weight of the food intake or of the complete food. The raw material is preferably included up to 1 % by weight in the diet. In case the food supplement also contains carriers and/ or excipients, the food supplement can be added up to 1.5% by weight relative to the total weight of the dry matter food intake or of the complete food, wherein the amount of raw larch material should not exceed 1 % by weight of the dry matter food intake or of the complete food. Cat and dog food is mostly provided in form of complete food comprising small uniform pieces, therefore, the larch wood material is preferably pulverized.

In a further aspect, the food material or the food supplement is administered to humans, and can be in the form of an ingredient designed to be added to a food product during manufacture at a rate of from 0.1 to 1.5% by weight relative to the total weight of the food product. In case the food supplement also contains carriers and/or excipients, the food supplement can be added up to 1.5% by weight relative to the total weight of the food product, wherein the amount of raw larch material should not exceed 1 % by weight of food product. The larch wood material is preferably pulverized. The food product can be solid or liquid and is preferably provided in form of cereal bars, cereal flakes, bread or shakes. With respect to shakes, the dry components (i.e. the wood material plus optionally other dry nutritional components such as sugars and other carbohydrates, sweeteners, nutritional proteins, fats or electrolytes) are preferably prepacked as a powder to be solved in liquid food, e.g. water, milk, soy milk, juice or yoghurt. Although the addition of the wood material to a food product during manufacture is preferred, an administration of the wood material in form of tablets or capsules is possible as well.

The present invention is further demonstrated and illustrated by the following examples, yet wi thout being restricted thereto.

### EXAMPLE 1: Assessment and characterization of the anti-inflammatory activity of extracts from larch sawdust in vitro.

### Material

Sawdust from *Larix decidua* (Pinaceae) (provided by Jannach Lärchenholz GmbH., Thalheim, Styria)

### Preparation of extracts

### 1.) Heptane extract

10 parts of powdered larch sawdust (mesh size 1 mm) were mixed with 100 parts n-heptane and stirred for 2 hours at ambient temperature. The extract was filtered and the solvent was evaporated at 40°C under reduced pressure. A yellowish oily residue was received (yield 0.50 %).

### 2.) 70% ethanol extract

10 parts of powdered larch sawdust (mesh size 1 mm) were mixed with 100 parts 70% ethanol (v/v) and stirred for 2 hours at ambient temperature. The extract was filtered and the solvent was evaporated at 40°C under reduced pressure (yield 1.45%).

### Isolation and structure elucidation of diterpene from the larch heptane extract

13 g of larch heptane extract were subjected to VLC on a silica gel column (8x7 cm), using a hexane- ethyl acetate gradient. Fraction 6 (hexane/ethyl acetate 90/10; 1,40 g) was further separated on Sephadex LH20® (60x2.8 cm; mobile phase: ethyl acetate), and isopimaric acid and palustric acid were isolated from purified fractions by semipreparative HPLC on a Thermo Hypercarb column (150x10 mm, 7 µm; mobile phase: acetonitrile/5% tetrahydrofurane isocratically, flow rate 4 ml/min). Fraction 7 (hexane/ethyl acetate 85:15, 2,36 g) was further purified on Sephadex LH20® (60x2.8 cm; mobile phase: ethyl acetate). Larixyl acetate, dehydroabietinol and dehydroabietic acid were isolated from purified fractions by semipreparative HPLC on a LiChrospher 100 RP18 column (250x10 mm, 7 µm; mobile phase: acetonitrile/water 65/35-90/10 in 30'; flow rate: 2 ml/min. Fraction 9 (hexane/ethyl acetate 70:30, 0,90 g) was purified using the same protocol as for fraction 7 to afford larixol. Structures of the pure compounds were elucidated by 1D and 2D NMR and mass spectrometry, and spectroscopic data were compared to literature data.

In vitro tests for COX-1, COX-2 and LT biosynthesis inhibitory activity

### 1.) LT biosynthesis inhibition assay

The assay was performed as described in [Michael Adams, Olaf Kunert, Ernst Haslinger, Rudolf Bauer: Inhibition of Leukotriene Biosynthesis by Quinolone Alkaloids from the Fruits of Evodia Rutaecarpa; Planta med. 2004 (70): 904-908] with some modifications.
*Principle:* Activated neutrophile granulocytes with 5-LOX activity are incubated with a defined concentration of test sample and arachidonic acid. After stopping the enzymatic reaction by addition of formic acid and centrifugation to remove cellular fragments, the produced LTB4 is quantified in the supernatant by means of an LTB4 EIA kit.
*Isolation of human neutrophile granulocytes:* 30 ml of venous human blood from healthy voluntary donors are collected with a VacutainerTM (BD, Plymouth, UK) system containing a 0.219 M pre-analytical citric acid solution. The blood is immediately transferred to a falcon tube containing 20 ml of sedimentation solution (1% NaCl, 6% dextran T-500) and left to separate for 60 min at 4°C. While most of the dense erythrocytes sink into the dextran layer, the lighter blood fractions remain in the upper layer which is then removed and centrifuged at 1600 rpm (4°C, 10 min) to concentrate the leukocytes. The plasma supernatant is discarded, the pellet is resuspended in 10 ml of a wash buffer (10% CaCl2.2H2O p.a.; 0.1% anhydrous d-glucose; 0.2%MgC12.6H2O; 0.04% KCl; 1.75% TRIS p.a.; with the pH adjusted to 7.6 with 1N HCl). After centrifugation at 1400 rpm (4°C, 10 min) and removal of the supernatant the resulting pellet is resuspended in 10 ml of a hypotonic lysis buffer (0.17% NH4Cl; 0.2% TRIS; pH 7.2) and gently shaken for 5 min at room temperature to destroy remaining erythrocytes. The suspension is submitted to another centrifugation at 1400 rpm (4°C, 5 min). The pellet is resuspended in 10 ml of wash buffer and then centrifuged at 1400 rpm (4°C, 15 min). The resulting pellet which now mainly contains neutrophile granulocytes is resuspended in 2 ml of TRIS buffer (1.75% TRIS p.a., 0.9% NaCl, pH 7.4), tested for vitality, quantified and then diluted to a cell concentration of 4500 cells/ml with TRIS buffer.
*Cell vitality test:* 50µl of cell suspension and 10 µl of trypan blue solution (0.4%) (Sigma Chemical Co. Steinheim, Germany) are mixed on a glass object carrier and examined with a light microscope at 1000 fold magnification. Dead cells appear larger and darker due to absorption of trypan blue. The vitality of the cells must be >95%.
*Determination of cell concentration:* 10µl of granulocyte cell suspension are dyed using 990 µl aqueous TÜRKS solution (Merck) and quantified in a "Neubauer" chamber (Assistent, Germany).
*Sample preparation:* samples are dissolved in an appropriate amount of ethanol p.a. to be added to the test mixture resulting in screening concentrations of 50 µM for pure compounds and 20µg/ml for crude. The commercially available 5-LOX inhibitor zileuton (Sequoia Research Products Ltd., UK) is used as a positive control at a concentration of 10 µM representing its IC50 in this test system.
*Incubation procedure:* The incubation mixture in a 96-well flat bottom microtitre plate (Bibby Sterilin, Staffordshire, UK) consists of 265 µl leukocyte suspension, with 75.5 µM CaCl2, 0.152 µM eicosatetraenoic acid (ETYA) as an inhibitor of the 12-LOX pathway, 0.642 µM Ca ionophore A23187 and 4.43 µM arachidonic acid, the substrate of the 5-LOX pathway, along with 5 µl of each test solution or 5 µl of EtOH p.a. in control wells. After incubation for 10 min at 37°C in darkness, the reaction is stopped by addition of 20 µl of 10% formic acid. The microtitre plate is centrifuged for 15 min at 1400 rpm to separate free LTB4 from cellular particles before the supernatant is diluted 40 fold in EIA buffer and applied to a competitive LTB4 EIA kit (Assay Designs Inc.).
LTB4 *EIA:* The plate is incubated for 2 hours at room temperature on an orbital shaker. After the EIA kit has been emptied and rinsed five times with EIA wash buffer it is developed with 200 µl substrate reagent in each well for 2 hours at 37°C. Hereby the substrate gains a distinct yellow color with an intensity directly proportional to the amount of LTB4 tracer bound to the well and thus inversely proportional to the amount of free LTB4 present in the well after incubation, enabling the quantification of LTB4 biosynthesis inhibition by measuring the absorption at 405 nm using a photometric ELISA plate reader after addition of 50 µl stop solution. The inhibition is expressed in percent in relation to a control experiment using only the solvent ethanol.

The final ethanol concentration of 1.89% in the control wells has no cytotoxic effects on the cells as could be shown by determination of the cell vitality by dying with trypan blue solution after the incubation to mark dead cells. At this concentration ethanol shows no inhibition of leukotriene biosynthesis. This was proven by using 5 µl of test buffer instead of ethanol and comparing the LTB4 concentrations. Inhibition values are means of at least 2 experiments and each sample is tested in duplicate. IC₅₀ values of active samples were determined by testing at least 3 concentrations in 3 different experiments, in duplicate each. IC₅₀ was calculated by logarithmic regression after graphical representation.

### 2.) COX-1 and COX-2 assays [B.L. Fiebich, M. Grozdeva, S. Hess, M. Hüll, U. Danesch, A.Bodensieck, R. Bauer; Planta Med. 2005 (71), 12-19]

*Principle:* The purified enzymes COX-1 and COX-2 are incubated with a defined concentration of test sample and arachidonic acid. After stopping the enzymatic reaction by addition of formic acid and dilution, the produced PGE2 is quantified by means of a PGE2 EIA kit. *Sample preparation:* The test samples (extracts and pure compounds) are dissolved in ethanol. Extracts are tested in concentrations of 20 µg/ml for screening. Pure compounds are tested in concentrations of 50 µM for screening.
*Incubation procedure:* The assays are performed in a microtiter scale with purified PGHS-1 (COX-1) from ram seminal vesicles and purified PGHS-2 (COX-2) from sheep placental cotyledones (both: Cayman Chemical Company, Ann Arbor, MI, USA). The incubation mixture consists of 180 µl 0.1 M TRIS/HCl- buffer (pH 8.0) (Roth, Karlsruhe, Germany), 5µM hematin, (porcine, ICN, Aurora, Ohio, USA), 18 mM epinephrine hydrogen tartrate (Fluka, Buchs, Switzerland), 0.2 U of enzyme preparation and 50µM Na2EDTA (only COX-2 assay, Titriplex III, Merck, Darmstadt, Germany). 10µl of each test solution are added and the mixture is preincubated for 5 min at room temperature. The reaction is started by adding 10µl of 5µM arachidonic acid (Cayman Chemical Company) in EtOH p.a. and the reaction mixture is incubated at 37°C. The reaction is terminated after 20 min by addition of 10 µl of 10% formic acid.
*PGE2 EIA:* The concentration of PGE2, the main arachidonic acid metabolite in this reaction, is determined by a competitive PGE2 EIA kit (Assay Designs Inc., Ann Arbor, MI, USA). All samples are diluted in EIA buffer.The EIA is incubated for 2 hours at room temperature on an orbital shaker. After a washing step, 200 µl of substrate solution are added to each well, and the EIA is incubated for 1 hour at room temperature. After addition of 50 µl stop solution, the EIA is evaluated photometrically by an ELISA microplate reader.

Inhibition of COX refers to reduction of PGE2 formation in comparison to a blank run without inhibitor. Inhibition values are means of at least 2 experiments and each sample is tested in duplicate. Indomethacin (ICN), NS-398 and nimesulide (both Cayman Chemical Company) are used as positive controls.. IC₅₀ values of active samples were determined by testing at least 3 concentrations in 3 different experiments, in duplicate each. IC₅₀ was calculated by logarithmic regression after graphical representation.

### 3.) Results: COX-1, COX-2 and LT biosynthesis inhibitory activity of larch extracts

It could be shown that two extracts produced from larch sawdust, namely a 70% ethanolic and a heptane extract, possess an inhibitory activity against COX-1, COX-2 and 5-LOX mediated LT bisoynthesis, wherein the inhibitory activity of the heptane extract was remarkably pronounced. (see table 1 - Inhibitory activity at the screening concentration (20µg/ml]).

The heptane extract from sawdust of *Larix decidua* MILL. was highly effective in all three assays, with IC₅₀ values of 5.0 µg/ml against COX-1, 0.1 µg/ml against COX-2 and 11.1 µg/ml against 5-LOX mediated LT formation(see table 2 - IC₅₀ values of highly active extracts (> 70 % inhibition at the screening concentration)). The 70% ethanol extract strongly inhibits COX-2 (IC₅₀ 0.8 µg/ml) (see Table 2). For comparison, the IC₅₀ value of the positive control in the COX-1 assay (indomethacin) is 0.3 µg/ml, in the COX-2 assay (NS-398) 0.8 µg/ml and in the LT assay (Zileuton) 1,2 µg/ml (see Table 3). The degree of activity of larch extracts is comparable to the activity of the aforementioned compounds, i.e. indomethacin, NS-398 and Zileuton, which are already established as therapeutics against inflammatory disorders and asthma.

**Table 1: Inhibitory activity at the screening concentration (20 µg/ml)**

| Extract | COX-1 | COX-2 | LT assay |
|---|---|---|---|
| Ethanol | 45,7±12,7% | 83,9±2,1% | 10,5±5,2% |
| n-heptane | 74,4±56,0% | 90,2±2,7% | 72,7±9,7% |

**Table 2: IC₅₀ values of highly active extracts (> 70 % inhibition at the screening concentration):**

| Extract | Enzyme | IC₅₀ |
|---|---|---|
| | COX-1 | 5,0 µg/ml |
| n-heptane | COX-2 | 0,1 µg/ml |
| | LT assay | 11,1 µg/ml |
| Ethanol | COX-2 | 0,8 µg/ml |

**Table 3: IC₅₀ values of the positive controls used**

| Compound | Enzyme | IC₅₀ |
|---|---|---|
| Indomethacin | COX-1 | 0,3 µg/ml |
| NS-398 | COX-2 | 0,8 µg/ml |
| Zileuton | LT assay | 1,2 µg/ml |

### 4.) Results: COX-1, COX-2 and LT biosynthesis inhibitory activity of larch diterpenes

Diterpenes isolated from the highly active larch sawdust heptane extract have shown to exhibit LT biosynthesis inhibitory activity to different degrees and moderate COX-2 inhibitory activity (see Tables 4 and 5; for positive controls see Table 6):
Larixyl acetate proved to be highly active against LT biosynthesis (IC₅₀ 10.4 µM), whereas it only possessed weak COX-2 inhibitory activity (IC₅₀ 95.0 µM) and was devoid of COX-1 inhitory activity. The structurally similar compound larixol was inactive against all three enzymes.
The abietane-type diterpene dehydroabietinol showed high LT formation inhibitory activity (IC₅₀ 10,9 µM) and was inactive against COX-1 and COX-2. The LT biosynthesis inhibitory activity of dehydroabietic acid was less pronounced (IC₅₀ 44,6 µM), which is in good agreement with published data showing moderate 5-LOX inhibitory activity for the similar compound abietic acid isolated from the cones of *Abies nordmanniana ssp. equitraojani* (IC₅₀ 29.5 ± 1.29 µm) [Ulusu et al. 2002. Phytotherapy Research 16:88-90]
Isopimaric acid proved to be a potent inhibitor of 5-LOX mediated LT biosynthesis (IC₅₀ 10.2 µM) without inhibiting COX-1 or COX-2.
The LT biosynthesis inhibitory potential of palustric acid was less pronounced (IC₅₀ 17.8 µM), however, this compound also possessed moderate COX-2 inhibitory activity (IC₅₀ 57,9 µM) without inhibiting COX-1.
Some of these diterpenes are known to possess antimicrobial, anti-ulcer and cardiovascular activities.

**Table 4: Screening of isolated diterpenes for COX-1, COX-2 and LT biosynthesis inhibitory activity (screening concentration 50 µM):**

| Compound | % COX-1 inhibition | %COX-2 inhibition | % LT inhibition |
|---|---|---|---|
| 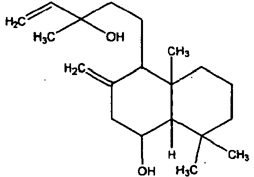 | 2,1±4,7 | 8,7±3,8 | 32,7±5,0 |
| Larixol | | | |
| 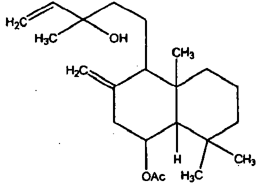 | 0,2±5,9 | 38,5±5,9 | 98,1±0,1 |
| larixyl acetate | | | |
| 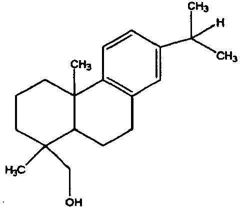 | -1,6±4,6 | 7,7±8,0 | 94,5±1,4 |
| dehydroabietinol | | | |
| 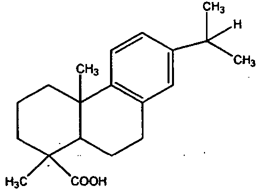 | 2,9±7,8 | -6,0±9,0 | 47,9±8,4 |
| dehydroabietic acid | | | |
| 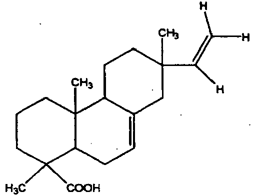 | 15,7±7,1 | 3,5±6,2 | 98,1±0,5 |
| isopimaric acid | | | |
| 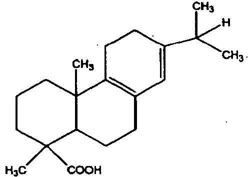 | 13,5±4,7 | 49,5±3,5 | 66,7±3,1 |
| palustric acid | | | |

**Table 5: IC₅₀ determination of compounds possessing pronounced activity at the screening concentration:**

| Compound | IC₅₀ COX-1 | IC₅₀ COX-2 | IC₅₀ LT assay |
|---|---|---|---|
| Larixol | Inactive | Inactive | >125 µM |
| Larixyl acetate | Inactive | 95.1 µM | 10.4 µM |
| Dehydroabietinol | Inactive | Inactive | 10.9 µM |
| Dehydroabietic acid | Inactive | Inactive | 44.6 µM |
| Isopimaric acid | Inactive | Inactive | 10.2 µM |
| Palustric acid | Inactive | 57.9 µM | 17.8 µM |

**Table 6: IC₅₀ of positive controls used:**

| Enzyme | Positive control | IC₅₀ |
|---|---|---|
| COX-1 | Indomethacin | 0.9 µM |
| COX-2 | NS-398 | 2.6 µM |
| LT assay | Zileuton | 5.0 µM |

Tables 7 and 8 illustrate the amount (% of the dry extract including the standard deviation (S.D.)) of larixyl acetate (LXA) and dehyroabietic acid (DHA) present in three different batches of heptane extracts and in two different batches of raw larch wood material (larch sawdust). The contents were characterized with HPLC.

**Table 7: Larixyl acetate and dehydroabietic acid in three different batches of the heptane extract:**

| Batches heptane extract | % DHA of the dry extract ±S.D. | % LXA of the dry extract ±S.D |
|---|---|---|
| Batch 1 | 3,60±0,02 | 6,32±0,02 |
| Batch 2 | 4,21±0,23 | 10,35±0,23 |
| Batch 3 | 3,42±0,02 | 6,32±0,02 |

**Table 8: Larixyl acetate and dehydroabietic acid in two different batches of larch sawdust**

| Batches larch sawdust | % DHA of the raw material ±S.D | % LXA of raw material ±S.D |
|---|---|---|
| Batch 1 | 0,022±0,001 | 0,056±0,002 |
| Batch 2 | 0,012±0,001 | 0,055±0,003 |

In summary, the good in vitro COX-1, COX-2 and 5-LOX inhibitory activity which was observed in larch ethanol and heptane extracts can partly be explained by the presence of diterpenes (particularly larixyl acetate), which have been isolated from the heptane extract, and turned out to be highly potent LT biosynthesis inhibitors or dual inhibitors of COX-2 and LT biosynthesis in *vitro.* These findings suggest administering the diterpene compounds in form of the raw larch wood material in order to treat inflammatory conditions.

### EXAMPLE 2: Application of larch sawdust as an ingredient in sow feed for treatment and prophylaxis of inflammation.

*Introduction:* The body's early defense in response to trauma, inflammation or infection, the acute phase response (APR), is a complex set of systemic reactions seen shortly after exposure to a triggering event. The APR is induced by protein hormones called cytokines acting as messengers between the local site of injury and the hepatocytes synthesing the acute phase proteins such as serum amyloid A. Most cytokines have multiple sources, targets and multiple functions. The proinflammatory cytokines can be divided into two major groups with respect to acute phase proteins inductions, the Interleukin-1 (IL-1) type (including Tumor Necrosis Factor-α, TNF-α) and the IL-6 type cytokines (including IL-6 cytokine). These cytokines are secreted primarily by monocytes activated by bacterial toxins or in response to local tissue injury.
The "Postpartum Dysgalactia Syndrome" (PPDS) causes important economical losses in the global swine industry. The aetiology of syndrome includes endotoxins and generally negative, as well as some positive gram bacteria (colibacteroides, β-haemolytic streptococci G and L, staphylococci, Arcanobacterium pyogenes, Proteus, Bacteroides, Clostridium and Haemophilus). Moreover, several aetiological agents of functional hypoagalactia, factors associated with stress of sows and conditions that contribute in the proliferation of bacteria and consequently in the potential endotoxemia (e.g. cystitis, metritis, vaginitis, constipation, mastitis), seem to play a significant role. Risk factors that are often suspected for PPDS are the health status of sows (Fat Sow Syndrome extended duration of parturition, post-partum pyrexia, teat malformation and injuries, as well as hypoplasia of mammary glands), the housing and management conditions of the sow around parturition, as well as diet composition. The clinical signs are characterized mainly by disorders of lactation and health status of sows (anorexia, depression, pyrexia, constipation and abnormal postpartum vulval discharge), as well as from decreased litter performance (unsuccessful attempts for suckling, intense discomposure, diarrhoea, poor growth rates, unevenness of litters regarding to body weight of piglets, increase of preweaning mortality).

*Principle:* Evaluation of the antiinflammatory activity of Larch sawdust in order to reduce the prevalence of Postpartum Dysgalactia Syndrome in sows under field conditions. IL-6 and TNF-α were analyzed as markers of inflammatory stress.

*Materials and Methods:* Sows with no clinical signs of disease were used from a farm of approximately 900 sows under production. Trial animals were sows of the same genotype (Dalland hybrids), chosen in terms of achieving homogenity regarding parity, bodyweight, previous production parameters (litter size, litter average bodyweight etc.) and previous health status. The farm was selected due to previous history of Post Partum Dysgalactia Syndrome occurrence. The sows were randomly allocated to a control group (n=5), a positive control group (n=5) and a larch sawdust (n=5) group. The whole trial period was 35 days beginning at 7 days prior to farrow until the day of weaning.
1% by weight of larch sawdust was added as an anti-inflammatory ingredient to sow feed (dry period and lactation feed). When it was necessary, such as with the larch sawdust, the tested material was grounded and filtered, so that at least 95% of the material's particles had a size of approximately 1 mm in order to provide good resorption of the possible anti-inflammatory compounds present in the compound by the body as well as an homogeneous distribution of the raw material in the feed. The raw materials were well accepted at an inclusion level of 1 % by weight in the diet as it was shown by previous trials performed.

Thus, during the whole trial period the animals were fed as follows:

| | |
|---|---|
| 1. Control group | (99% Basic diet and 1 % Corn starch) |
| 2. Larch group | (99% Basic diet and 1 % Larch) |
| 3. Positive control group | (99% Basic diet and 1% Corn starch) |

Moreover, the animals of the positive control group were injected a single 2 ml dosage of an antiinflammatory drug (meloxicam) at the day of parturition, while the animals of all the other groups were injected with a single 2 ml dose of normal saline at the same day.

Blood samples were collected from all trial animals at allocation day (7 days before farrowing day) and at 24 and 72 hours post farrowing from all sows for inflammation markers evaluation (serum TNF-α, plasma IL-6). Additionally the milk production index for all sows was calculated, while production and health parameters of sows and their litters were recorded.

The cytokines results were expressed as n-fold increase of cytokine expression in relation to untreated animals at allocation day.

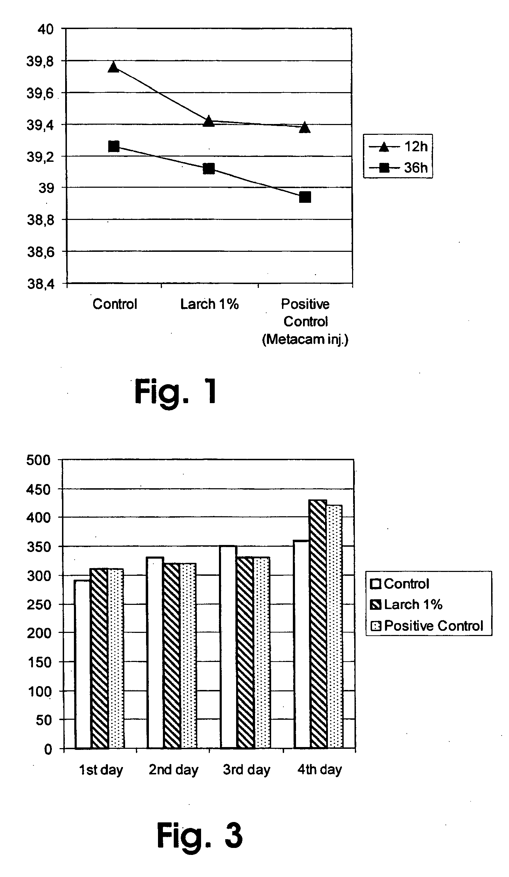

*Results:* Concerning the larch sawdust evaluation, the respective results have shown that sows feeding with this agent resulted in a significant decrease of serum IL6 and plasma TNFα concentrations 24 hours postpartum (see Table 9). Additionally, an improvement tendency in production and health parameters was revealed comparatively to the control group (see Figs.1, 2a, 2b and 3).

**Table 9.Relative expression (n fold) of IL6 and TNFα of sows after 24 h and 96 h Post Partum (PP) in relation to untreated animals at allocation day.**

| Sampling | Group | IL6 | TnFα |
|---|---|---|---|
| 24 hours PP | Controls | 4.05±1.07 | 3.67±1.62 |
| | Larch 1% | 2.96±1.02 | 2.11±0.71* |
| | Positive controls | 1.86±0.92* | 0.87±0.29* |
| 72 hours PP | Controls | 2.48±1,71 | 2.68±1.32 |
| | Larch 1 % | 1.59±0.86 | 1.07±0.55* |
| | Positive controls | 1.08±0.78 | 0.98±0.77* |

| | | | |
|---|---|---|---|
| **P* < *0.05 significantly different from* C *group* | | | |

The mean rectal temperatures (°C) of sows 12 and 36 hours post farrowing is shown in Fig.1.

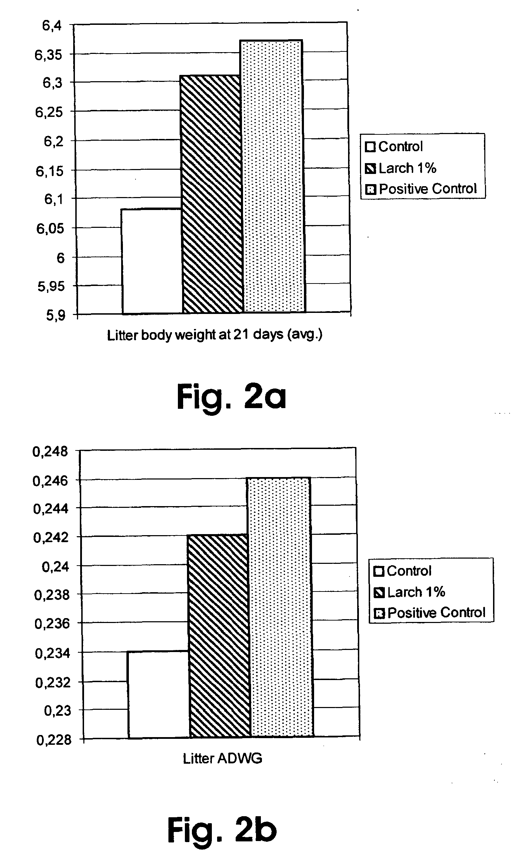

The Average litter body weight at 21 days of age (kg) is shown in Fig. 2a and the relative average litter body weight gain (kg) is shown in Fig. 2b for the control group, the larch sawdust group and the positive control group.

Average milk lactation index 1, 2, 3 and 4 days post farrowing is shown in Fig. 3.

### References:

Gabay C., Kushner .: Acute-phase proteins and other systemic responses to inflammation. N. Engl. J. Med. 340:448-454,1999.
Mackiewicz A.: Acute phase proteins and transformed cells. Int. Rev. Cytol, 170:225-300, 1999.
Papatsiros V.G., Alexopoulos C., Kyriakis: S.C. Latest information in relation to Postpartum Dysgalactia Syndrome of sows. J. Hellenic Vet. Med. Soc., 58:61-75, 2007.
Suffredini A.F., Fantuzzi G., Badolato R., Oppenheim J.J., O'Grady N.: New insights into the biology of the acute phase response. J. Clin. Immunol.,19: 203-214, 1999.

### EXAMPLE 3: Application of larch sawdust as an ingredient in ruminants feed for treatment and prophylaxis of inflammation.

*Short summary of the experiment:* Two levels, 3.3% (low dose) and 10% (high dose), of larch sawdust is added as an anti-inflammatory ingredient to ruminants feed (dry period). The larch sawdust is grounded and filtered, such that at least 95% of the wood particles have a size up to 3 mm but not less than 0.5 mm resulting in an homogeneous distribution of the raw material in the feed. Experimental protocol for feeding trial and anti-inflammatory activity: Larch anti-inflammatory activity is tested in three groups of 5 heifers each. One group is fed with a ration at maintenance and 1 kg of dry beet pulp. The other two groups are fed with the same ration, providing that 0.3 and 0.9 kg/d of dry beet pulp is substituted by 0.3 and 0.9 kg/d of larch. After a 21 days adaptation period, heifers are injected with ACTH-analogous for 5 days, to stimulate cortisol secretion, simulating a pro-inflammatory status. Parameters recorded: blood sampling and analysis of mRNA expression of proinflammatory cytokines (tumor necrosis factor alpha, plasma interleukin-6, interleukin-2 and interferon-gamma). Results: Feeding trials with ruminants (cattle) indicated that larch sawdust can be included until 10% dry matter of daily ration without affecting intake and rumen activity. A good anti-inflammatory effect in cattle was observed at the low and the high dose.

*Introduction:* Psychological and physiological stress (environmental stress) stimulates the hypothalamus-pituitary-adrenal (HPA) axis, sympathetic nervous system, and immune system. These systems interact each other, leading to the complex stress response. Corticotropin-releasing hormone, adrenocorticotropic hormone, and glucocorticoids stimulate production of cytokines and modify inflammatory and immune responses. Cytokines are regulatory polypeptides that are produced by different cell types in the presence of appropriate stimuli (infectious and not infectious factors). Mayor cytokines involved in the initiation of inflammation are tumor necrosis factor α (TNF-α), interleukin 1 (IL-1) and IL-6. These cytokines are released in the early stages of an immune response from a variety of cell types, including activated immune cells, such as macrophages, vascular endothelial cells, fibroblasts, and neurons and have different actions: activation of arachidonic acid metabolism, priming of polymorphonuclear leukocytes (PMN), up-regulation of adhesion molecules and induction of other cytokines. Another group of cytokines is that which mediates later, adaptive immunity, such as the T cell cytokines IL-2 and IFN-γ (type II interferons), which are especially important in mediating anti-viral defenses.

Principle: Activation of the SPA axis by administering exogenous ACTH has been used to investigate the influence of Larch sawdust on stressed cows. Cytokine gene expression changes were analyzed as markers of inflammatory stress.

*Animals and sample collection:* Fifteen dairy Friesian heifers, homogeneous for age and body conditions, with no clinical signs of disease were used. The heifers were housed together and randomly allocated to a control group (CTR; n = 5) and two compound treatment group (High; Low; n = 5). During the trial period, animals were fed twice a day a basal diet (concentrate and forage) plus the addition, in the morning meal, of 1 kg of dehydrated beet pulp and larch sawdust in the ratio 1.0:0.0 (CRT); 0.1:0.9 (HIGH); 0.7:0.3 (LOW) respectively in CTR, High and Low groups. After 21 days, all heifers were given 0.5 mg of ACTH (Synachten, Novartis, Varese) every 12 hours for 5 consecutive days. Blood was collected from jugular vein at the morning, before (day 18 and day 21) and during the ACTH challenge (day 23=T1 and day 25=T2; 1 hour after ACTH injection).

*Leucocytes isolation and RNA extraction:* Blood samples (5ml) were mixed with 5 volumes of lysis buffer EL (Qiagen) and centrifuged at 400 x g for 10' at 4°C. Pellet was resuspended in 2 volumes of buffer EL, centrifuged at 400 x g for 10' at 4°C, washed with cold sterile phosphate buffer saline (PBS) and then stored at -80°C in 1 ml of TRIZOL (Invitrogen, Milan, Italy). Total RNA was isolated according to the manufacture's instructions of TRIZOL Reagent (Invitrogen, Milan Italy). Absorbance measurements at 260nm and 280nm were used to assess RNA sample concentration and purity (A260 : A280 > 1.7).

*Reverse transcription and Real-time SybrGreen PCR:* The cDNA was synthesized on RNA samples (1000ng) by using Improm-II Reverse Transcriptase (Promega, Milan Italy) according to the manufacturer's instructions. The concentration of cDNA was assumed as 50ng/µl. Target genes were IFNγ (interferon-gamma), TNFα (tumour necrosis factor), IL-2 (Interleukin-2), IL-6 (interleukin-6); housekeeping (HKG) genes were SDHA (succinate dehydrogenase complex subunit A), and BACT (beta-actin). Specific oligonucleotide primers were designed from GenBank sequence information with the help of Primer3 Input software (Rozen and Skaletsky, 2000). Real-time RT-PCR reactions were performed in triplicate using Platinum®. SYBR® Green qPCR SuperMix-UDG (Invitrogen, Milan, Italy). For amplifications, 45 cycles were applied (1 s at 95°C, 30 s at specific annealing temperature, 30 s at 72°C) in a 96-well spectrofluorometric thermal cycler (DNA Engine Opticon 2; MJ Research, Inc.Waltham, MA USA). Finally, melting curve analysis of amplification products was performed. Product specific PCR cycle conditions were deduced by designing standard curves consisting of serial dilution of the appropriate cDNA. Amplification efficiency (E) was determined from the slope of standard curves as E=10-1/slope. The expression data were computed by the 2^{-ΔΔCt} method (Bustin, 2000; Pfaffl, 2001), where ΔCt = (C_{targer}-Ct_{NF}) and Ct_{NF} is the geometrical mean of Ct of housekeeping genes of each sample. The raw data of gene expression (ΔCt) of each heifer at day 18 and day 21 were averaged and referred to as T0. The levels of gene regulation at 48 (T1) and 96 (T2) hours of ACTH challenge in each experimental group (CTR, High, Low) relatively to T0, were calculated as nfold = 2^-^{(ΔCt-Tx - ΔCt-T0)} for upregulated genes, and as nfold =-2^(^{ΔCt-Tx - ΔCt-T0)} for down-regulated genes.

*Results:* Larch sawdust caused a significant inhibition of ACTH-stimulated upregulation of IFNγ, IL2, IL6 and TNFα (see Table 10). At T1, Low dose of Larch diminished IL2 upregulation of 23.9% *vs* CTR group, whereas High dose diminished IL6 upregulation of 29.9%. At T2 the effect of Larch was more evident. In the Low group, the ACTH-induced stimulation of IFN, IL2 and TNF resulted lower than in CTR group of 37,33.8 and 66.4% respectively. In the High group the ACTH-induced stimulation of IFN, IL2 resulted lower than in CTR group of 45.5 and 41.4% respectively.

**Table 10. Relative expression (nfold) of IFNγ, IL2, IL6 and TNFα in heifers after 48 h (T1) and 96 h (T2) of ACTH challenge, compared to basal conditions.**

| Sampling | Group | IFNγ | IL2 | IL6 | TNFα |
|---|---|---|---|---|---|
| T1 | Ctr | 2.3 ± 0.6 | 3.3 ± 0.4 | 3.4 ± 0.6 | 0.5 ± 0.8 |
| | Low | 1.5 ± 1.3 | 2.5* ± 0.8 | 2.8 ± 0.8 | 0.7 ± 1.0 |
| | High | 1.8 ± 0.8 | 2.9 ± 0.9 | 2.4* ± 0.5 | 0.5 ± 0.6 |
| T2 | Ctr | 2.7 ± 0.3 | 3.4 ± 0.2 | 2.5 ± 1.1 | 1.2 ± 0.3 |
| | Low | 1.7** ± 0.5 | 2.3** ± 0.4 | 2.1 ± 0.5 | 0.4* ± 0.4 |
| | High | 1.5* ± 0.9 | 2.0* ± 1.0 | 1.3 ± 0.7 | 1.3 ± 0.2 |

| | | | | | |
|---|---|---|---|---|---|
| *P < 0.05, ** P < 0.005 significantly different from CTR group | | | | | |

### References:

Bustin, S.A., 2000. Absolute quantification of mRNA using real-time reverse transcription polymerase chain reaction assays. J. Mol. Endocrinol. 25,169-193.
Pfaffl, M.W., 2001. A new mathematical model for relative quantification in real-time RT-PCR. Nucleic Acids Res. 29, e45.
Rozen, S., Skaletsky, H.J., 2000. Primer3 on the WWW for general users and for biologist programmers. Bioinformatics Methods and Protocols: Methods in Molecular Biology. Humana Press, 365-386.

## Claims

1. Use of raw wood material from a tree of the genus *Larix* in the manufacture of a medicament for treatment or prophylaxis of inflammation in humans or animals.

2. The use according to claim 1 relating to an inflammation measurable in blood.

3. The use according to claim 1 or 2 relating to an inflammation associated with an infectious disease.

4. The use according to claim 1 or 2 relating to a non-infectious inflammation.

5. The use according to claim 4, **characterized in that** the non-infectious inflammation is selected from the group consisting of inflammation associated with environmental stress, inflammation occurring in cows around parturition ("transition cows") and inflammation associated with osteoarthrosis.

6. The use according to any of the claims 1 to 5, **characterized in that** the wood material is reduced to small pieces, wherein at least 95% by weight has a size less than or equal to 3 mm.

7. The use according to claim 6, **characterized in that** at least 95% by weight of the wood material has a size between 0.5 and 1.5 mm, inclusively.

8. The use according to claim 6, **characterized in that** the wood material is pulverized, wherein at least 95% by weight has a size between 0,05 and 0,5 mm, inclusively.

9. The use according to any of the claims 1 to 8, **characterized in that** the wood material is larch sawdust.

10. The use according to any of the claims 1 to 9 in a food supplement, **characterized in that** the wood material represents up to 100% by weight of the food supplement, preferably 70 - 99 % by weight in said food supplement, wherein the residual portion represents carriers and/or excipients.

11. The use according to any of the claims 1 to 9, **characterized in that** the wood material is an anti-inflammatory ingredient in a nutritional composition.

12. The use according to any of the claims 1 to 11, **characterized in that** the wood material is administered to ruminants, and that it is added to a food ration or a daily food intake given to ruminants or embedded during the manufacture of a complete food at a rate of up to 10%by weight, preferably 2-5% by weight, relative to the total weight of the food intake or of the complete food.

13. The use according to any of the claims 1 to 11, **characterized in that** the wood material is administered to non-ruminant, herbivore farm animals, particularly rabbits and horses, and that it is added to a food ration or a daily food intake given to non-ruminant, herbivore farm animals or embedded during the manufacture of a complete food at a rate of up to 1,5% by weight, preferably at 1% by weight, relative to the total weight of the food intake or of the complete food.

14. The use according to any of the claims 1 to 11, **characterized in that** the wood material is administered to non-herbivore farm animals and domestic animals, particularly swine, poultry, cats, dogs and rodents, and that it is added to a food ration or a daily food intake given to the animals or embedded during the manufacture of a complete food at a rate of up to 1% by weight relative to the total weight of the food intake or of the complete food.

15. The use according to any of the claims 1 to 11, **characterized in that** the wood material is administered to humans, and that it is added to a food product during manufacture at a rate of up to 1% by weight relative to the total weight of the food product.

## Patentansprüche

1. Verwendung von Rohholzmaterial von einem Baum der Gattung *Larix* zur Herstellung eines Medikamenrs zur Behandlung oder zur Prophylaxe einer Entzündung beim Menschen oder beim Tier.

2. Verwendung nach Anspruch 1 bezogen auf eine im Blut messbare Entzündung.

3. Verwendung nach Anspruch 1 oder 2 bezogen auf eine Entzündung, die mit einer Infektionskrankheit in Zusammenhang steht.

4. Verwendung nach Anspruch 1 oder 2 bezogen auf eine nichtinfcktiöse Entzündung.

5. Verwendung nach Anspruch 4, **dadurch gekennzeichnet, dass** die ruchtinfektiöse Entzündung ausgewählt ist aus der Gruppe bestehend aus einer Entzündung in Zusammenhang mit umgebungsbedingter Sttessbelastung, einer Entzündung, die bei Kühen um den Zeitpunkt des Abkalbens auftritt ("transition cows"), und einer Entzündung in Zusammenhang mit Osreoarthrose.

6. Verwendung nach einem der Ansprüche 1 bis 5, **dadurch gekennzeichnet, dass** das Holzmaterial zerkleinert ist, wobei zumindest 95 Gew.-% eine Größe von kleiner oder gleich 3 mm aufweisen.

7. Verwendung nach Anspruch 6, **dadurch gekennzeichnet, dass** zumindest 95 New.-% des Holzmaterials eine Größe zwischen 0,5 und 1,5 mm einschließlich aufweisen.

8. Verwendung nach Anspruch 6, **dadurch gekennzeichnet, dass** das Holzmaterial pulverisiett ist, wobei zumindest 95 Gew.-% eine Größe zwischen 0,05 und 0,5 mm einschließlich aufweisen.

9. Verwendung nach einem der Ansprüche 1 bis 8, **dadurch gekennzeichnet, dass** es sich bei dem Holzmaterial um Lärchensägemehl handelt.

10. Verwendung nach einem der Ansprüche 1 bis 9 in einem Nahrungsergänzungsmittel, **dadurch gekennzeichnet, dass** das Holzmaterial bis zu 100 Gew.% des Nahrungserganzungsmittels ausmacht, vorzugsweise 70 - 99 Gew.-% des Nahrungsezgänzungsmittels, wobei der Rest durch Träger und/oder Hilfsstoffe gebildet ist.

11. Verwendung nach einem der Ansprüche 1 bis 9, **dadurch gekennzeichnet, dass** das Holzmaterial ein entzündungshemmender Restandteil in einer Nahrzusammensetzung ist.

12. Verwendung nach einem der Ansprüche 1 bis 11, **dadurch gekennzeichnet, dass** das Heizmaterial Wiederkäuern verabreicht wird und dass es einer Nahrungsration oder einer täglichen Nahrungsaufnahme, die Wiederkäuern gegeben wird, hinzugefügt oder während der Herstellung einer Vollnahrung eingebettet wird, in Höhe von bis zu 10 Gew.-%, vorzugsweise 2-5 5 Gew.-%, in Bezug auf das Gesamtgewicht der Nahrungsaufnahme oder der Vollnahrung

13. Verwendung nach einem der Ansprüche 1 bis 11, **dadurch gekennzeichnet, dass** das Holzmaterial nichrwiederkäuenden, herbivoren Nutztieren, insbesondere Kaninchen und Pferden, verabreicht wird und dass es einer Nahrungsration oder einer täglichen Nahrungsaufnahme, die nichtwiederkäuenden, herbivoren Nutztieren gegeben wird, hinzugefügt oder während der Herstellung einer Vollnahrung eingebettet wird, in Höhe von bis zu 1,5 Gew.-%, vorzugsweise 1 Gew.-%, in Bezug auf das Gesamtgewicht der Nahrungsaufnahme oder der Vollnahrung.

14. Verwendung nach einem der Ansprüche 1 bis 11, **dadurch gekennzeichnet, dass** das Holzmaterial nichtherbivoren Nutztieren und Haustiere, insbesondere Schweinen, Geflügel, Katzen, Hunden und Nagern, verabreicht wird und dass es einer Nahrungstation oder einer täglichen Nahrungsaufnahme, die den Tieren gegeben wird, hinzugefügt oder während der Herstellung einer Vollnahrung eingebettet wird, in Höhe von bis zu 1 Gew.-% in Bezug auf das Gesamtgewicht der Nahrungsaufnahme oder der Vollnahrung.

15. Verwendung nach einem der Ansprüche 1 bis 11, **dadurch gekennzeichnet, dass** das Holzmaterial Menschen verabreicht wird, und dass es einem Nahrungsmittelprodukt während der Herstellung in Höhe von bis zu 1 Gew.-% in Bezug auf das Gesamtgewicht des Nahrungsmittelprodukts hinzugefügt wird.

## Revendications

1. Utilisation de matériau de bois brut provenant d'un arbre du genre *Larix* dans la fabrication d'un médicament pour le traitement ou la prophylaxie d'une inflammation chez les humains ou les animaux.

2. Utilisation selon la revendication 1, liée à une inflammation mesurable dans le sang.

3. Utilisation selon la revendication 1 ou 2, liée à une inflammation associée à une maladie infectieuse.

4. Utilisation selon la revendication 1 ou 2, liée à une inflammation non-infectieuse.

5. Utilisation selon la revendication 4, **caractérisée en ce que** l'inflammation non-infectieuse est choisie dans le groupe constitué d'une inflammation associée à un stress environnemental, une inflammation survenant chez les vaches au moment de la parturition (« vaches en transition ») et l'inflammation associée à l'arthrose.

6. Utilisation selon l'une quelconque des revendications 1 à 5, **caractérisée en ce que** le matériau de bois est réduit en petits morceaux, dans laquelle au moins 95 % en poids ont une taille inférieure ou égale à 3 mm.

7. Utilisation selon la revendication 6, **caractérisée en ce qu'**au moins 95 % en poids du matériau de bois ont une taille comprise entre 0,5 et 1,5 mm, inclus.

8. Utilisation selon la revendication 6, **caractérisée en ce que** le matériau de bois est pulvérisé, dans laquelle au moins 95 % en poids ont une taille comprise entre 0,05 et 0,5 mm, inclus.

9. Utilisation selon l'une quelconque des revendications 1 à 8, **caractérisé en ce que** le matériau de bois est de la sciure de mélèze.

10. Utilisation selon l'une quelconque des revendications 1 à 9 dans un complément alimentaire, **caractérisée en ce que** le matériau de bois représente jusqu'à 100 % en poids du complément alimentaire, de préférence 70-99 % en poids dudit complément alimentaire, dans laquelle la partie résiduelle représente des supports et/ou excipients.

11. Utilisation selon l'une quelconque des revendications 1 à 9, **caractérisée en ce que** le matériau de bois est un ingrédient anti-inflammatoire dans une composition nutritionnelle.

12. Utilisation selon l'une quelconque des revendications 1 à 11, **caractérisée en ce que** le matériau de bois est administré à des ruminants, et qu'il est ajouté à une ration alimentaire ou à une prise alimentaire journalière donnée à des ruminants ou intégrée pendant la fabrication d'un aliment complet à un taux pouvant atteindre 10 % en poids, de préférence 2-5 % en, poids, par rapport au poids total de la prise alimentaire ou de l'aliment complet.

13. Utilisation selon l'une quelconque des revendications 1 à 11, **caractérisée en ce que** le matériau de bois est administré à des animaux de ferme non-ruminants, herbivores, en particulier des lapins et des chevaux, et qu'il est ajouté à une ration alimentaire ou à une prise alimentaire journalière donnée à des animaux de ferme non-ruminants, herbivores, ou intégrée pendant la fabrication d'un aliment complet à un taux pouvant atteindre 1,5 % en poids, de préférence à 1 % en poids, par rapport au poids total de la prise alimentaire ou de l'aliment complet.

14. Utilisation selon l'une quelconque des revendications 1 à 11, **caractérisée en ce que** le matériau de bois est administré à des animaux de ferme non-herbivores et à des animaux domestiques, en particulier des porcs, la volaille, des chats, des chiens et des rongeurs, et qu'il est ajouté à une ration alimentaire ou à une prise alimentaire journalière donnée aux animaux ou intégrée pendant la fabrication d'un aliment complet à un taux pouvant atteindre 1 % en poids par rapport au poids total de la prise alimentaire ou de l'aliment complet.

15. Utilisation selon l'une quelconque des revendications 1 à 11, **caractérisée en ce que** le matériau de bois est administré aux humains, et qu'il est ajouté à un produit alimentaire pendant la fabrication à un taux pouvant atteindre 1 % en poids par rapport au poids total du produit alimentaire.
